(19) Europäisches Patentamt
European Patent Office
Office européen des brevets

(11) **EP 2 160 241 B1**

(12) **EUROPÄISCHE PATENTSCHRIFT**

(45) Veröffentlichungstag und Bekanntmachung des Hinweises auf die Patenterteilung:
**12.04.2017 Patentblatt 2017/15**

(21) Anmeldenummer: **08758963.6**

(22) Anmeldetag: **02.06.2008**

(51) Int Cl.:
**B01J 37/02** *(2006.01)* **B01J 23/22** *(2006.01)*
**B01J 23/881** *(2006.01)* **B01J 23/888** *(2006.01)*

(86) Internationale Anmeldenummer:
**PCT/EP2008/004398**

(87) Internationale Veröffentlichungsnummer:
**WO 2008/145405 (04.12.2008 Gazette 2008/49)**

(54) **VERFAHREN ZUM AUFTRAGEN EINER WASHCOATSUSPENSION AUF EINE TRÄGERSTRUKTUR**

METHOD FOR APPLYING A WASH COAT SUSPENSION TO A CARRIER STRUCTURE

PROCÉDÉ DESTINÉ À L'APPLICATION D'UNE SUSPENSION D'ENDUIT LAVÉ SUR UNE STRUCTURE SUPPORT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.05.2007 DE 102007025357**

(43) Veröffentlichungstag der Anmeldung:
**10.03.2010 Patentblatt 2010/10**

(73) Patentinhaber: **Süd-Chemie IP GmbH & Co. KG**
**80333 München (DE)**

(72) Erfinder:
• **MESTL, Gerhard**
**80935 München (DE)**
• **GÜCKEL, Christian**
**85567 Grafing (DE)**

• **ESTENFELDER, Marvin**
**85560 Ebersberg (DE)**
• **KÄDING, Bastian**
**21018 Sesto Calende (IT)**

(74) Vertreter: **Stolmár & Partner**
**Patentanwälte PartG mbB**
**Blumenstraße 17**
**80331 München (DE)**

(56) Entgegenhaltungen:
**WO-A-2005/030380 WO-A-2006/027009**
**DE-A1- 19 709 589 DE-A1-102005 055 827**
**US-A- 4 259 211**

Bemerkungen:
Die Akte enthält technische Angaben, die nach dem Eingang der Anmeldung eingereicht wurden und die nicht in dieser Patentschrift enthalten sind.

EP 2 160 241 B1

**Beschreibung**

Die vorliegende Erfindung betrifft ein Verfahren zur Herstellung von Schalenkatalysatoren durch Auftragen einer Washcoatsuspension auf eine Trägerstruktur.

Schalenkatalysatoren und Verfahren zu deren Herstellung sind im Stand der Technik bekannt. Bestimmte Schalenkatalysatoren werden beispielsweise hergestellt, indem eine ein pulverförmiges Trägeroxid sowie eine katalytisch wirksame Spezies enthaltende Washcoatsuspension auf eine Trägerstruktur in Form einer Schale aufgetragen wird. Mit Schalenkatalysatoren ist in vielen Fällen eine selektivere Reaktionsführung möglich als mit Katalysatoren, die bis in den Kern der Trägerstruktur hinein mit der katalytisch aktiven Spezies beladen sind.

Phthalsäureanhydrid (PSA) beispielsweise wird gegenwärtig überwiegend mittels Schalenkatalysatoren in hoher Selektivität hergestellt. Der überwiegende Anteil der gegenwärtig eingesetzten Schalenkatalysatoren zur Herstellung von PSA sind Schalenkatalysatoren mit einer $V_2O_5/TiO_2$-umfassenden Schale auf einem nicht-porösen, als Hohlzylinder ausgebildeten Steatitträger. In dem $V_2O_5/TiO_2$-System dieser Katalysatoren liegt das Aktivmetalloxid $V_2O_5$ vermutlich nicht nur in Form von kristallinen $V_2O_5$-Partikeln vor. Derartige Schalenkatalysatoren werden üblicherweise durch Beschichtung mittels entsprechender Suspensionen in Dragiertrommeln oder Wirbelschichtanlagen hergestellt.

Vorrichtungen zum Mischen, Trocknen und Beschichten von pulverigem, körnigem oder geformtem Schüttgut in einem Reaktorbett und ein Verfahren zur Herstellung von Trägerkatalysatoren, insbesondere Schalenkatalysatoren, unter Verwendung einer solchen Vorrichtung, waren bekannt (WO 2005/030380). Ebenfalls bekannt waren Verfahren zum Aufbringen einer Suspension auf einen Trägerkörper im Fließbettverfahren (DE 10 2005 055827, US-A-4 259 211). Die vorgenannten Techniken sind jedoch an die Grenzen angelangt, was die Homogenität der Schichtdicke von entsprechend hergestellten Schalenkatalysatoren angeht, und zwar sowohl was die Homogenität der Schichtdicke eines einzelnen Schalenkatalysators angeht als auch die einer Charge von Schalenkatalysatoren.

Aufgabe der vorliegenden Erfindung ist es daher ein Verfahren bereitzustellen, mittels welchem Beschichtungen mit weitgehend einheitlicher Dicke ausgehend von Washcoatsuspensionen auf eine Trägerstruktur aufgetragen werden können.

Diese Aufgabe wird durch ein Verfahren unter Nutzung einer Vorrichtung gelöst, die eingerichtet ist, mittels eines Prozessgases ein Fließbett von Trägerstrukturen zu erzeugen, in welchem die Trägerstrukturen toroidal umlaufen, umfassend die Schritte des

a) Beschickens der Vorrichtung mit Trägerstrukturen und Erzeugens eines Trägerstruktur-Fließbettes mittels eines Prozessgases, wobei die Trägerstrukturen in dem Fließbett toroidal umlaufen;

b) Imprägnierens der Trägerstrukturen mit einer Washcoatsuspension durch Besprühen der in dem Fließbett toroidal umlaufenden Trägerstrukturen mit der Washcoatsuspension;

c) Trocknens der mit der Washcoatsuspension besprühten Trägerstrukturen;

d) gegebenenfalls, Kalzinierens der mit den Feststoffanteilen der Washcoatsuspension beladenen Trägerstrukturen,

worin die Vorrichtung (10) eine Prozesskammer (15) mit einem Boden (16) umfasst, in dessen Mitte eine Ringspaltdüse (50) angeordnet ist, die Prozesskammer (15) ferner eine Seitenwand (18) umfasst, wobei das Prozessgas (40) durch den Boden (16) der Prozesskammer (15), der vorzugsweise aus mehreren übereinander gelegten, sich einander überlappenden ringförmigen Leitplatten (25, 26, 27, 29) aufgebaut ist, und wobei die äußere Leitplatte (25) zu der Seitenwand (18) beabstandet ist, zwischen denen ringförmige Schlitze (28) ausgebildet sind, mit einer horizontalen, radial nach außen gerichteten Bewegungskomponente in die Prozesskammer (15) eingeführt wird zur Erzeugung des Trägerstruktur-Fließbettes, und worin dem in die Prozesskammer (15) eingeführten Prozessgas (40) eine umfängliche Strömungskomponente auferlegt wird, indem durch den Boden (16) der Prozesskammer (15) zusätzliches Prozessgas (61) mit einer schräg nach oben gerichteten Bewegungskomponente in die Prozesskammer (15) im Bereich der Seitenwand (18) der Prozesskammer (15) eingeführt wird.

Überraschenderweise wurde festgestellt, dass mittels des erfindungsgemäßen Verfahrens Trägerstrukturen mit einer weitgehend einheitlichen Schichtdicke beschichtet werden können.

Washcoatsuspensionen sind im Stand der Technik bekannt. Dabei handelt es sich um Suspensionen, die zumindest ein Metalloxid (z.B. $Al_2O_3$, $TiO_2$, $ZurO_2$ usw.) in partikulärer Form enthalten. Ebenso können Mischungen verschiedener Metalloxide eingesetzt werden. Die Metalloxide werden gewöhnlich in einem Suspensionsmittel, zumeist Wasser, dispergiert und dann auf einen Träger aufgebracht. Die Aufbringung erfolgt meist durch Tauch- oder Sprühverfahren. Zusätzlich können noch weitere Zuschlagstoffe, wie Binder, Füllmittel, Stabilisatoren, Promotoren oder dergleichen in der Suspension enthalten sein. Während und/oder nach der Aufbringung der Washcoat-Suspension wird diese getrocknet und gegebenenfalls kalziniert. Je nach Prozesseinsatz des Coat-Katalysators kann der Washcoat in nachfolgenden Schritten beispielsweise mit oder mit weiterem katalytisch aktivem Material imprägniert werden, beispielsweise durch Tauchen, Sprühen, Imprägnieren und dergleichen, wobei im Anschluss gewöhnlich weitere Trocknungs- und gegebenenfalls Kalzinier- und/oder Reduktionsschritte erfolgen können.

In dem erfindungsgemäßen Verfahren wird ein Fliessbett erzeugt, in welchem die Trägerstrukturen toroidal umlaufen. Im Stand der Technik wird der Übergang der Partikel einer Schüttung in einen Zustand, in welchen die Partikel vollständig frei beweglich werden (Fließbett), als Lockerungspunkt (Wirbelpunkt) bezeichnet und die entsprechende Fluidgeschwindigkeit als Lockerungsgeschwindigkeit. Erfindungsgemäß bevorzugt ist es, dass in dem erfindungsgemäßen Verfahren die Fluidgeschwindigkeit bis zum 4-fachen der Lockerungsgeschwindigkeit beträgt, bevorzugt bis zum drei-fachen der Lockerungsgeschwindigkeit und mehr bevorzugt bis zum 2-fachen der Lockerungsgeschwindigkeit. Gemäß einer alternativen Ausführungsform des erfindungsgemäßen Verfahrens kann es vorgesehen sein, dass die Fluidgeschwindigkeit bis zum 1,4-fachen des Zehnerlogarithmus der Lockerungsgeschwindigkeit beträgt, bevorzugt bis zum 1,3-fachen des Zehnerlogarithmus der Lockerungsgeschwindigkeit und mehr bevorzugt bis zum 1,2-fachen des Zehnerlogarithmus der Lockerungsgeschwindigkeit.

Die Trocknung der mit der Suspension besprühten Trägerstrukturen erfolgt im Rahmen des erfindungsgemäßen Verfahrens vorzugsweise kontinuierlich mittels des Prozessgases. Es kann aber auch vorgesehen sein, dass nach Imprägnierung unter kontinuierlicher Trocknung ein gesonderter abschließender Trocknungsschritt durchgeführt wird. Im ersten Fall, beispielsweise, kann durch die Temperatur des Prozessgases bzw. der Trägerstrukturen die Trocknungsgeschwindigkeit und damit beispielsweise die Gleichmäßigkeit der Dicke der Schale eingestellt werden, im zweiten Fall kann durch jede dem Fachmann als geeignet bekannte Trocknungsmethode getrocknet werden.

Vorrichtungen zur Durchführung des erfindungsgemäßen Verfahrens sind beispielsweise in den Dokumenten WO 2006/027009 A1, DE 102 48 116 B3, EP 0 370 167 A1, EP 0 436 787 B1, DE 199 04 147 A1, DE 20 2005 003 791 U1 beschrieben. Weitere geeignete und erfindungsgemäß bevorzugte Fließbettanlagen werden beispielsweise von den Unternehmen Glatt GmbH (Binzen, Deutschland), Aeromatic-Fielder AG (Bubendorf, Schweiz), Fluid Air Inc. (Aurora, Illinois, USA), Oystar Hüttlin GmbH (Schopfheim, Deutschland), Umang Pharmatech Pvt. Ltd. (Marharashtra, Indien) und Innojet Technologies (Steinen, Deutschland) vertrieben.

Zur Durchführung des erfindungsgemäßen Verfahrens besonders bevorzugte Fließbettvorrichtungen werden von der Firma Innojet Technologies unter den Bezeichnungen Innojet® Ventilus oder Innojet® Air-Coater vertrieben. Diese Vorrichtungen umfassen einen zylindrischen Behälter mit einem fest und unbeweglich eingebauten Behälterboden, in dessen Mitte eine Sprühdüse montiert ist. Der Boden besteht aus kreisrunden Lamellen, die stufenweise übereinander montiert sind. Die Prozessluft strömt zwischen den einzelnen Lamellen waagerecht exzentrisch mit einer umfänglichen Strömungskomponente nach außen in Richtung der Behälterwand in den Behälter ein. Dabei bilden sich so genannte Luftgleitschichten aus, auf denen die Trägerstrukturen zunächst nach außen in Richtung Behälterwand transportiert werden. Außen an der Behälterwand wird ein senkrecht ausgerichteter Prozessluftstrom geführt, der die Trägerstrukturen nach oben umlenkt. Oben angekommen bewegen sich die Trägerstrukturen auf einer mehr oder weniger tangentialen Bahn in Richtung Mitte des Bodens zurück, in dessen Verlauf sie den Sprühnebel der Düse passieren. Nach dem Passieren des Sprühnebels beginnt der beschriebene Bewegungsvorgang von Neuem. Die beschriebene Prozessluftführung liefert dabei die Grundlage für eine weitgehend homogene, toroidale fließbettartige Umwälzbewegung der Trägerstrukturen.

Das Zusammenwirken des Besprühens mit der fließbettartigen torusförmigen Umwälzbewegung der Trägerstrukturen im Fließbett bewirkt im Gegensatz zu einer entsprechenden herkömmlichen Wirbelschicht, dass die einzelnen Trägerstrukturen in annähernd gleicher Häufigkeit die Sprühdüse passieren. Darüber hinaus sorgt der Umwälzvorgang auch dafür, dass die einzelnen Trägerstrukturen eine Rotation um ihre eigene Achse durchführen, weshalb die Trägerstrukturen besonders gleichmäßig beschichtet werden.

In dem erfindungsgemäßen Verfahren laufen die Trägerstrukturen in dem Fließbett toroidal um. Um eine Vorstellung davon zu geben, wie sich die Strukturen in dem Fließbett bewegen sei ausgeführt, dass sich bei "elliptischem Umlaufen" die Trägerstrukturen in dem Fließbett in vertikaler Ebene auf einer elliptischen Bahn bewegen mit wechselnder Größe der Haupt- und Nebenachse. Bei "toroidalem Umlaufen" bewegen sich die Trägerstrukturen in dem Fließbett in vertikaler Ebene auf einer elliptischen Bahn mit wechselnder Größe der Haupt- und Nebenachse und in horizontaler Ebene auf einer Kreisbahn mit wechselnder Größe des Radius. Im Mittel bewegen sich die Trägerstrukturen bei "elliptischem Umlaufen" in vertikaler Ebene auf einer elliptischen Bahn, bei "toroidalem Umlaufen" auf einer toroidalen Bahn, d.h., dass eine Trägerstruktur die Oberfläche eines Torus mit vertikal elliptischem Schnitt helikal abfährt.

Gemäß dem erfindungsgemäßen Verfahren ist es vorgesehen, dass die Vorrichtung eine Prozesskammer -zur Aufnahme von Trägerstrukturen- mit einem Boden umfasst, in dessen Mitte eine Ringspaltdüse angeordnet ist.

Zur Bewerkstelligung eines Trägerstruktur-Fließbettes, in welchem die Trägerstrukturen toroidal umlaufen, auf verfahrenstechnisch einfache und damit kostengünstige Weise, kann gemäß der vorliegenden Offenbarung die Prozesskammer eine Seitenwand umfassen, wobei das Prozessgas durch den Boden der Prozesskammer, der vorzugsweise aus mehreren übereinandergelegten, sich einander überlappenden ringförmigen Leitplatten aufgebaut ist, zwischen denen ringförmige Schlitze ausgebildet sind, mit einer horizontalen, radial nach außen gerichteten Bewegungskomponente in die Prozesskammer eingeführt wird.

Dadurch, dass Prozessgas mit einer horizontalen, radial nach außen gerichteten Bewegungskomponente in die Prozesskammer eingeführt wird, wird ein elliptisches Umlaufen der Trägerstrukturen in dem Fließbett bewirkt. Sollen die Strukturen in dem Fließbett toroidal umlaufen, so muss den Trägerstrukturen zusätzlich noch eine umfängliche Bewegungskomponente auferlegt werden, welche die Strukturen auf eine Kreisbahn zwingt. Diese umfängliche Bewegungskomponente kann den Strukturen beispielsweise auferlegt werden, indem an der Seitenwand entsprechend ausgerichtete Führungsschienen zur Umlenkung der Trägerstrukturen angeordnet sind. Gemäß dem erfindungsgemäßen Verfahren ist es jedoch vorgesehen, dass dem in die Prozesskammer eingeführten Prozessgas eine umfängliche Strömungskomponente auferlegt wird. Dadurch wird die Erzeugung des Fließbettes, in welchem die Träger toroidal umlaufen, verfahrenstechnisch einfach gewährleistet.

Erfindungsgemäß ist es vorgesehen sein, dass dem in die Prozesskammer eingeführten Prozessgas die umfängliche Strömungskomponente auferlegt wird, indem durch den Boden der Prozesskammer zusätzliches Prozessgas mit einer schräg nach oben gerichteten Bewegungskomponente in die Prozesskammer im Bereich der Seitenwand der Prozesskammer eingeführt wird. Um dem in die Prozesskammer eingeführten Prozessgas die umfängliche Strömungskomponente aufzuerlegen, kann es gemäß einer bevorzugten Ausführungsform des erfindungsgemäßen Verfahrens vorgesehen sein, dass zwischen den ringförmigen Leitplatten entsprechend geformte und ausgerichtete Prozessgas-Leitelemente angeordnet sind.

Es kann vorgesehen sein, dass das Besprühen der in dem Fließbett umlaufenden Strukturen mit der Suspension mittels der Ringspaltdüse durchgeführt wird, indem diese eine Sprühwolke versprüht, wobei die Symmetrieebene der Sprühwolke parallel zur Ebene des Gerätebodens verläuft oder die Sprühwolke schräg nach oben ausgerichtet ist. Durch den 360°-Umfang der Sprühwolke können die sich mittig herabbewegenden Trägerstrukturen besonders gleichmäßig mit der Lösung besprüht werden. Dabei ist die Ringspaltdüse, d.h. deren Mündung, vorzugsweise vollständig in dem Fließbett eingebettet. Durch die vorzugsweise schräg nach oben ausgerichtete Sprührichtung wird die Prozessluft des torodialen Fließbetts unterstützt.

Wie bereits vorstehend ausgeführt, ist es entsprechend einer weiter bevorzugten Ausführungsform des erfindungsgemäßen Verfahrens vorgesehen, dass die Ringspaltdüse mittig im Boden des Behälters angeordnet ist und die Mündung der Ringspaltdüse in dem Fließbett vollständig eingebettet ist. Dadurch wird gewährleistet, dass die freie Weglänge der Tropfen der Sprühwolke bis zum Auftreffen auf einen Träger verhältnismäßig kurz ist und entsprechend den Tropfen verhältnismäßig wenig Zeit verbleibt, zu größeren Tropfen zu koaleszieren, was der

Ausbildung einer weitgehend einheitlichen Schalendicke entgegenwirken könnte.

Gemäß einer weiter bevorzugten Ausführungsform des erfindungsgemäßen Verfahrens kann es vorgesehen sein, dass an der Unterseite der Sprühwolke ein Gasstützpolster bewerkstelligt wird. Das bodenseitige Polster hält die Bodenoberfläche weitgehend frei von versprühter Suspension, das heißt, dass nahezu die gesamte versprühte Suspension in das Fließbett eingetragen wird, so dass kaum Sprühverluste auftreten.

Gemäß einer weiter bevorzugten Ausführungsform des erfindungsgemäßen Verfahrens kann es vorgesehen sein, dass die Trägerstrukturen aus einem nicht-porösen (kompakten) Material oder Materialgemisch gebildet ist, vorzugsweise aus Steatit, Quarz ($SiO_2$), Porzellan, Magnesiumoxid, Zinndioxid, Siliciumcarbid, Rutil, Tonerde ($Al_2O_3$), Aluminiumsilicat, Magnesiumsilicat, Zirkoniumsilicat oder aus Cersilicat oder aus Mischungen von zwei oder mehr der vorstehenden Materialien.

Beim Umwälzen der Trägerstrukturen im Rahmen des erfindungsgemäßen Verfahrens werden diese mechanisch beansprucht, wodurch es zu einem gewissen Abrieb des bereits aufgecoateten Washcoats kommen kann. Insbesondere um den Abrieb der Strukturen in vertretbaren Grenzen zu halten, weist die Trägerstruktur eine Härte von größer/gleich 20 N auf, vorzugsweise eine von größer/gleich 30 N, weiter bevorzugt eine von größer/gleich 40 N, am meisten bevorzugt eine von größer/gleich 80 N und insbesondere 20 bis 150 N. Die Ermittlung der Härte ist dabei mittels eines Tablettenhärtetesters 8M der Fa. Dr. Schleuniger Pharmatron AG an 99 Stück Strukturen als Durchschnitt bestimmt nach Trocknung bei 130 °C für 2 h, wobei die Geräteeinstellungen wie folgt sind:

| | |
|---|---|
| Härte: | N |
| Distanz zum Formkörper: | 5,00 mm |
| Zeitverzögerung: | 0,80 s |
| Vorschub-Typ: | 6 D |
| Geschwindigkeit: | 0,60 mm/s |

Aus Kostengründen wird in dem erfindungsgemäßen Verfahren als Prozessgas vorzugsweise Luft eingesetzt. Es kann auch vorgesehen sein, dass als Prozessgas ein Inertgas eingesetzt wird, beispielsweise Stickstoff, Methan, kurzkettige gesättigte Kohlenwasserstoffe, eines der Edelgase, vorzugsweise Helium, Neon oder Argon, ein halogenierter Kohlenwasserstoff oder Mischungen davon.

Gemäß einer weiteren bevorzugten Ausführungsform des erfindungsgemäßen Verfahrens kann das Prozessgas, vor allem im Falle teurer Gase wie z.B. Helium, Argon etc., in einem geschlossenen Kreislauf in die Vorrichtung rückgeführt werden.

Gemäß einer weiter bevorzugten Ausführungsform des erfindungsgemäßen Verfahrens werden die Trägerstrukturen vor und/oder während des Auftragens der Suspension erwärmt, beispielsweise mittels eines erwärmten Prozessgases. Dabei wird entsprechend einer weiter bevorzugten Ausführungsform des erfindungsgemäßen Verfahrens das Prozessgas erwärmt, vorzugsweise auf eine Temperatur von größer/gleich 40 °C, bevorzugt auf eine Temperatur von größer/gleich 60 °C, weiter bevorzugt auf eine Temperatur von größer/gleich 70 °C und am meisten bevorzugt auf eine Temperatur von 60 bis 100 °C.

Um ein vorzeitiges Abtrocknen von Tropfen der Sprühwolke zu verhindern, kann es gemäß einer weiter bevorzugten Ausführungsform des erfindungsgemäßen Verfahrens vorgesehen sein, dass das Prozessgas vor der Einführung in die Vorrichtung mit dem Suspensionsmittel der Suspension angereichert wird, vorzugsweise in einem Bereich von 10 bis 50 % des Sättigungsdampfdrucks (bei Prozesstemperatur).

Gemäß einer weiteren bevorzugten Ausführungsform des erfindungsgemäßen Verfahrens kann das dem Prozessgas zugegebene Suspensionsmittel sowie Suspensionsmittel von der Abtrocknung der Strukturen mittels geeigneter Kühleraggregate, Kondensatoren und Abscheider vom Prozessgas abgetrennt und mittels einer Pumpe in den Suspensionsmittelanreicherer rückgeführt werden.

Es ist bevorzugt, dass die Trägerstruktur als Kugel, Zylinder, Lochzylinder, Trilobus, Tetralobus, Ring, Donut, Stern, Wagenrad oder als Strang, vorzugsweise als Rippstrang oder Sternstrang, ausgebildet ist.

Ferner ist es bevorzugt, dass die Washcoatsuspension $TiO_2$, $V_2O_5$ sowie $Sb_2O_3$ in partikulärer Form enthält. Zusätzlich oder alternativ dazu können in der Suspension auch Metalloxide wie z.B. $SiO_2$ $Al_2O_3$, $ZrO_2$ Zeolithe, $Cr_2O_3$, $MoO_3$, $WO_3$, $Nb_2O_5$, $Ta_2O_5$, $Fe_2O_3$ sein.

Weiter ist es bevorzugt, dass die Suspension ferner eine Phosphatverbindung, eine Alkali- oder Erdalkaliverbindung, z.B. Cäsiumverbindung sowie ein organisches und/oder anorganisches Bindemittel umfasst.

Auch ist es bevorzugt, dass die Washcoatsuspension auf einen Ring als Trägerstruktur aufgesprüht wird.

Darüber hinaus ist es bevorzugt, dass die Washcoatsuspension Eisenmolybdat enthält.

Ferner ist es bevorzugt, dass die Washcoatsuspension ein Mischoxid von Mo, V und W, oder Mo, V, und Nb, oder Ta sowie gegebenenfalls Te oder Sb enthält, das vorzugsweise mit Cu, Mn oder Fe dotiert ist.

Die vorliegende Erfindung betrifft ferner eine Charge einer Vielzahl von Schalenkatalysator hergestellt nach dem erfindungsgemäßen Verfahren, jeder Schalenkatalysator umfassend einen Formkörper als Trägerstruktur, auf dem eine ein metalloxidisches Material umfassende Schale aufgetragen ist, wobei das Verhältnis der Standardabweichung der Schalendicken der Schalenkatalysatoren der Charge zum Mittelwert der Schalendicken der Schalenkatalysatoren der Charge 3 bis 15 %. Derartige Schalenkatalysatoren sind mittels des erfindungsgemäßen Verfahrens erhältlich.

Die Schalendicke eines einzelnen Schalenkatalysators wird vorzugsweise als Mittelwert bestimmt, indem der Schalenkatalysator halbiert und die Schichtdicken an jeweils 4 um 90° versetzten Stellen unter dem Mikroskop bestimmt wird.

Die Standardabweichung ist dabei entsprechend der Formel

$$\sigma_X = \sqrt{\frac{1}{N-1}\sum_{i=1}^{N}(X_i - \overline{X})^2}$$

ermittelt, in welcher

$\sigma_x$ die Standardabweichung ist;
N (= 100) der Stichprobenumfang ist (Anzahl der Trägerstrukturen; N ist gleich 100);
$X_i$ die Schalendicke am i-ten Träger der Stichprobe ist;
X der empirische Mittelwert der Schalendicke der Stichprobe (also das arithmetische Mittel der Stichprobe) ist, der sich entsprechend der Formel

$$\overline{X} = \frac{1}{N}\sum_{i=1}^{N}X_i$$

bestimmt. Derartige Schalenkatalysator-Chargen sind mittels des erfindungsgemäßen Verfahrens herstellbar.

Ebenfalls beschrieben ist, dass die Vorrichtung eine Prozesskammer mit einem Boden umfasst, in dessen Mitte eine Ringspaltdüse angeordnet ist.

Weiterhin beschrieben ist, dass die Prozesskammer eine Seitenwand und einen Boden umfasst, wobei der Boden aus mehreren, übereinander gelegten, sich einander überlappenden, ringförmigen Leitplatten aufgebaut ist, zwischen denen ringförmige Schlitze ausgebildet sind, über die Prozessgas mit einer horizontalen, radial nach außen gerichteten Bewegungskomponente einführbar ist. Dadurch wird auf eine verfahrenstechnisch einfache Weise die Ausbildung eines Fließbettes ermöglicht, in welcher die Formkörper besonders gleichmäßig elliptisch oder toroidal umlaufen, was mit einer Steigerung der Produktqualität einhergeht.

Um ein besonders gleichmäßiges Besprühen der Strukturen zu gewährleisten, kann vorgesehen sein, dass die Mündung der Düse derart ausgebildet ist, dass mit dieser eine Sprühwolke versprühbar ist, deren horizontale Spiegelebene parallel zur Bodenebene verläuft.

Ferner ist beschrieben, dass mit der Düse eine Sprühwolke versprühbar ist, die schräg nach oben ausgerichtet ist, vorzugsweise ein Sprühwolke in Form eines nach oben ausgerichteten Sprühkegels, wobei dieser vorzugsweise die Prozessluft unterstützt.

Ferner kann es sein, dass zwischen der Mündung der Ringspaltdüse und dem darunterliegenden Boden Austrittsöffnungen für Stützgas vorgesehen sind, um an der Unterseite der Sprühwolke ein Stützpolster zu bewerkstelligen. Das bodenseitige Luftkissen hält die Bodenoberfläche frei von versprühter Suspension.

Entsprechend einer weiteren beschriebenen Verwendung wird in der Vorrichtung das Stützgas von der Ringspaltdüse selbst und/oder durch Prozessgas bereitgestellt. Diese Maßnahmen lassen sehr variable Ausgestaltungen der Bewerkstelligung des Stützgases zu. Es können an der Ringspaltdüse selbst Austrittsöffnungen vorgesehen sein, über die ein Teil des Sprühgases austritt, um zur Ausbildung des Stützgases beizutragen. Zusätzlich oder alternativ können Teile des Prozessgases, das durch den Boden strömt, in Richtung der Unterseite der Sprühwolke geführt werden und dadurch zur Ausbildung des Stützgases beitragen.

Entsprechend der Offenbarung weist die Ringspaltdüse einen kegelförmigen- oder hyperbolischen Kopf auf und die Mündung verläuft entlang einer kreisförmigen Kegelschnittfläche. Dadurch wird gewährleistet, dass durch den Kegel die sich vertikal von oben nach unten bewegenden Strukturen gleichmäßig und gezielt auf die Sprühwolke zugeführt werden, die vom kreisförmigen Sprühspalt am unteren Ende des Kegels versprüht wird.

Entsprechend der Offenbarung ist im Bereich zwischen Mündung und darunterliegendem Boden eine kegelstumpfförmige oder hyperbolische Wand vorgesehen, die vorzugsweise Durchtrittsöffnungen für Stützgas aufweist. Diese Maßnahme hat den Vorteil, dass die zuvor erwähnte harmonische Umlenkbewegung am Kegel durch die Fortsetzung über den Kegelstumpf aufrechterhalten wird und in diesem Bereich Stützgas durch die Durchtrittsöffnungen austreten kann und für die entsprechende Stützung an der Unterseite der Sprühwolke sorgt.

Gemäß der Offenbarung ist zwischen der Unterseite der kegelstumpfförmigen oder hyperbolischen Wand ein ringförmiger Schlitz zum Durchtritt von Prozessgas ausgebildet. Diese Maßnahme hat den Vorteil, dass der Übergang der Trägerstrukturen auf das Luftpolster des Bodens besonders gut gesteuert werden kann und unmittelbar im Bereich unter der Düse beginnend gezielt durchgeführt werden kann.

Um die Sprühwolke in gewünschter Höhe in das Fließbett eintragen zu können, ist es bevorzugt, dass die Lage der Mündung der Düse in der Höhe verstellbar ist.

Entsprechend der offenbarten Verwendung sind zwischen den ringförmigen Leitplatten Leitelemente angeordnet, die dem durchtretenden Prozessgas eine umfängliche Strömungskomponente auferlegen.

Die nachstehenden Beispiele dienen der Erläuterung der Erfindung.

Vergleichsbeispiel 1:

Phthalsäureanhydrid(PSA)-Katalysator

Zur Herstellung eines Katalysators mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem so genannten Wirbelbett-Coater (UnilabJ, Fa. Oystar Hüttlin, Schopfheim, Deutschland) 2600 g Steatitkörper in Form von Hohlzylindern der Größe $8 \times 6 \times 5$ mm mit einer Suspension aus 17,9 g Vanadiumpentoxid, 7,6 g Antimontrioxid, 1,28 g Cäsiumsulfat, 1,9 g Ammoniumdihydrogenphosphat, 364,4 g Titandioxid mit einer BET-Oberfläche von 26 m2/g, 130,5 g Bindemittel aus einer 50%-igen Dispersion von Wasser und Vinylacetat/Ethylencopolymer (EP 65, Fa. Air Products) und 1000 g Wasser beschichtet. Dazu wurde die Suspension auf ein mittels auf 80 °C temperierter Prozessluft erzeugtes Wirbelbett aufgesprüht.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 72 % aller gemessenen Formkörper hatten eine Schichtdicke von 158 $\mu$m.

Beispiel 1:

Es wurden Katalysatoren analog dem Vergleichsbeispiel 1 hergestellt, mit dem Unterschied, dass ein Fließbettcoater mit der Bezeichnung Innojet® Aicoater, Fa. Innojet, Steinen, Deutschland eingesetzt wurde, in welchem ein Fließbett erzeugt wurde, in welchem die Steatitkörper toroidal umliefen.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 90 % aller gemessenen Formkörper hatten eine Schichtdicke von 130 $\mu$m.

Vergleichsbeispiel 2:

Formaldehyd(FA)-Katalysator

Zur Herstellung eines Formaldehyd-Katalysators mit einem Aktivmassenanteil (Komponente a)) von 20 Gew.-%, einem Feststoffanteil eines organischen Bindemittels (Komponente b)) von 20 Gew.-% und einem Feststoffanteil einer anorganischen haftungsvermittelnden Komponente (Komponente(c)) von 1 Gew.-%, wurden in einem Wirbelbett-Coater (Unilab®, Fa. Oystar Hüttlin, Schopfheim, Deutschland) 700 g Steatitkörper (Dichte 2,7 g/cm$^3$) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus $Fe_2(MoO_4)_3$ und $MoO_3$, (hergestellt gemäß Beispiel 1 der EP 1 674 156 A1 (molares Verhältnis (Mo : Fe = 2,5))) werden unter Rühren suspendiert. Zur besseren Homogenisierung wird die Suspension 3 min auf Stufe 6 mit einem Ultra - Turrax® behandelt. Unter Rühren werden 9,22 g $ZrO_2$-Sol (20 % Feststoffgehalt, acetatstabilisiert, Fa. Nyacol, Handelsbezeichnung: NYACOL® Zirconia (Acetate)) zugegeben. Der pH-Wert der Suspension wird mit einer 25 %-igen Ammoniaklösung auf 4 eingestellt. Zu der Suspension werden 92 g des organischen Bindemittels (50 %-ige Dispersion von Wasser und Vinylacetat/Ethylencopolymer, EP 65, Fa. Air Products) gegeben und die Suspension unter Rühren eine Stunde homogenisiert.

Zur Beschichtung wurde die Suspension auf ein mittels auf 80 °C temperierter Prozessluft erzeugtes Wirbelbett aufgesprüht.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 74 % aller gemessenen Formkörper hatten eine Schichtdicke von 300 μm.

Beispiel 2:

Es wurden Katalysatoren analog dem Vergleichsbeispiel 2 hergestellt, mit dem Unterschied, dass ein Fließbettcoater mit der Bezeichnung Innojet® Aicoater, Fa. Innojet, Steinen, Deutschland eingesetzt wurde, in welchem ein Fließbett erzeugt wurde, in welchem die Steatitkörper toroidal umliefen.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 88 % aller gemessenen Formkörper hatten eine Schichtdicke von 250 μm.

Vergleichsbeispiel 3:

Acrylsäure-Katalysator

Zur Herstellung eines Katalysators mit einem Aktivmassenanteil (Komponente a)) von 20 Gew.-%, einem Feststoffanteil eines organischen Bindemittels (Komponente b)) von 20 Gew.-% und einem Feststoffanteil einer anorganischen haftungsvermittelnden Komponente (Komponente(c)) von 1 Gew.-%, wurden in einem Wirbelbett-Coater (Unilab®, Fa. Oystar Hüttlin, Schopfheim, Deutschland) 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml vollentsalztes Wasser vorgelegt. 184 g eines Aktivmassenpulvers ($Cu/MoVWO_x$) werden unter Rühren suspendiert. Zur besseren Homogenisierung wird die Suspension 3 min auf Stufe 6 mit dem Ultra - Turrax® behandelt. Unter Rühren werden 9,22 g eines $ZrO_2$-Sols (20 % Feststoffgehalt, acetatstabilisiert, Fa. Nyacol, Handelsname: NYACOL® Zirconia (Acetate)) dazugegeben. Der pH-Wert der Suspension wird mit einer 25 %-igen Ammoniaklösung auf 4 eingestellt. Zu der Suspension werden 92 g eines organischen Bindemittels (50 %-ige Dispersion von Wasser und Vinylacetat / Ethylencopolymer, EP 65, Fa. Air Products) gegeben und die Suspension unter Rühren eine Stunde homogenisiert.

Zur Beschichtung wurde die Suspension auf ein mittels auf 80 °C temperierter Prozessluft erzeugtes Wirbelbett aufgesprüht.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 72 % aller vermessenen Formkörper hatten eine Schichtdicke von 2480 μm.

Beispiel 3:

Es wurden Katalysatoren analog dem Vergleichsbeispiel 3 hergestellt, mit dem Unterschied, dass ein Fließbettcoater mit der Bezeichnung Innojet® Aicoater, Fa. Innojet, Steinen, Deutschland eingesetzt wurde, in welchem ein Fließbett erzeugt wurde, in welchem die Steatitkörper toroidal umliefen.

Es wurden 50 Hohlzylinder entnommen, halbiert und die Schichtdicken an jeweils 4 um 90° versetzten Stellen unter dem Mikroskop bestimmt. Die Schichtdicke eines Hohlzylinders entspricht dem Mittelwert der 4 gemessenen Werte. 92 % aller vermessenen Formkörper hatten eine Schichtdicke von 1680 μm.

Zeichnung:

Die nachstehende Beschreibung einer bevorzugten Vorrichtung zur Durchführung des erfindungsgemäßen Verfahrens sowie die Beschreibung von Bewegungsbahnen von Katalysatorträger-Formkörpern als Trägerstrukturen dient im Zusammenhang mit der Zeichnung der Erläuterung der Erfindung. Es zeigen:

Fig. 1A　　eine vertikale Schnittansicht einer bevorzugten Vorrichtung zur Durchführung des erfindungsgemäßen Verfahrens;

Fig. 1B　　eine Vergrößerung des in der Fig. 1A um-

rahmten und mit dem Bezugzeichen 1B markierten Bereichs;

Fig. 2A    eine perspektivische Schnittansicht einer nicht erfindungsgemäßen Vorrichtung, in welcher die Bewegungsbahnen zweier elliptisch umlaufender Katalysatorträger-Formkörper schematisch dargestellt sind;

Fig. 2B    eine Draufsicht auf die Vorrichtung und die Bewegungsbahnen gemäß Fig. 2A;

Fig. 3A    eine perspektivische Schnittansicht einer Vorrichtung, in welcher die Bewegungsbahn eines toroidal umlaufenden Katalysatorträger-Formkörpers schematisch dargestellt ist;

Fig. 3B    eine Draufsicht auf die Vorrichtung und die Bewegungsbahn gemäß Fig. 3A.

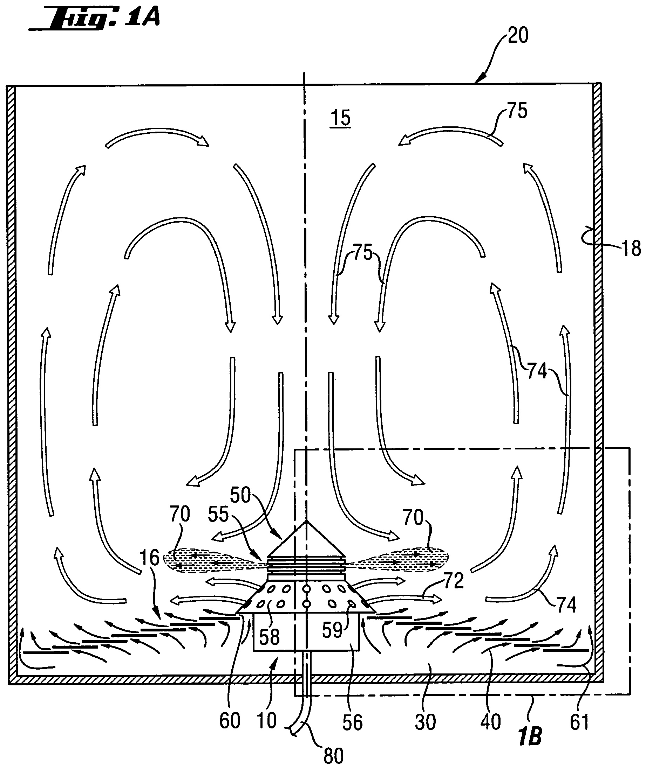

In der Fig. 1A ist eine insgesamt mit dem Bezugzeichen 10 belegte Vorrichtung zur Durchführung des erfindungsgemäßen Verfahrens gezeigt.

Die Vorrichtung 10 weist einen Behälter 20 mit einer aufrecht stehenden zylindrischen Seitenwand 18 auf, die eine Prozesskammer 15 umschließt.

Die Prozesskammer 15 weist einen Boden 16 auf, unter dem sich eine Anströmkammer 30 befindet.

Der Boden 16 ist aus insgesamt sieben ringförmigen übereinander gelegenen Ringplatten als Leitplatten zusammengesetzt. Die sieben Ringplatten sind so übereinander gesetzt, dass eine äußerste Ringplatte 25 eine unterste Ringplatte bildet, auf der dann die weiteren sechs inneren Ringplatten, die jeweils darunter liegende teilweise überlappend, aufgelegt sind.

Der Übersicht halber sind nur einige der insgesamt sieben Ringplatten mit Bezugszeichen versehen, beispielsweise die beiden übereinander liegenden Ringplatten 26 und 27. Durch diese Übereinanderlegung und Beabstandung ist zwischen zwei Ringplatten jeweils ein ringförmiger Schlitz 28 ausgebildet, durch den Prozessluft 40 als Prozessgas mit einer überwiegend horizontal gerichteten Bewegungskomponente durch den Boden 16 hindurch treten kann.

In der mittigen obersten inneren Ringplatte 29 ist in deren zentraler Öffnung von unten eine Ringspaltdüse 50 eingesetzt. Die Ringspaltdüse 50 weist eine Mündung 55 auf, die insgesamt drei Mündungsspalte 52, 53 und 54 aufweist. Alle drei Mündungsspalte 52, 53 und 54 sind so ausgerichtet, dass sie etwa parallel zum Boden 16, also etwa horizontal mit einem Umfassungswinkel von 360° aussprühen. Alternativ kann die Sprühdüse derart konstruiert sein, dass der Sprühkegel schräg nach oben verläuft. Über den oberen Spalt 52 sowie den unteren Spalt 54 wird Sprühluft als Sprühgas ausgepresst, durch den mittleren Spalt 53 die zu versprühende Suspension.

Die Ringspaltdüse 50 weist einen stabförmigen Körper 56 auf, der nach unten fortreicht und die entsprechenden Kanäle und Zuführleitungen enthält, die an sich bekannt und deshalb in der Zeichnung nicht dargestellt sind. Die Ringspaltdüse 50 kann beispielsweise mit einem so genannten rotativen Ringspalt ausgebildet sein, bei dem sich Wände des Kanals, durch den die Lösung ausgesprüht wird, relativ zueinander drehen, um Verstopfungen der Düse zu vermeiden, so dass über den Umfassungswinkel von 360° gleichmäßig aus dem Spalt 53 ausgesprüht werden kann.

Die Ringspaltdüse 50 weist oberhalb des Mündungsspalts 52 einen kegelförmigen Kopf 57 auf.

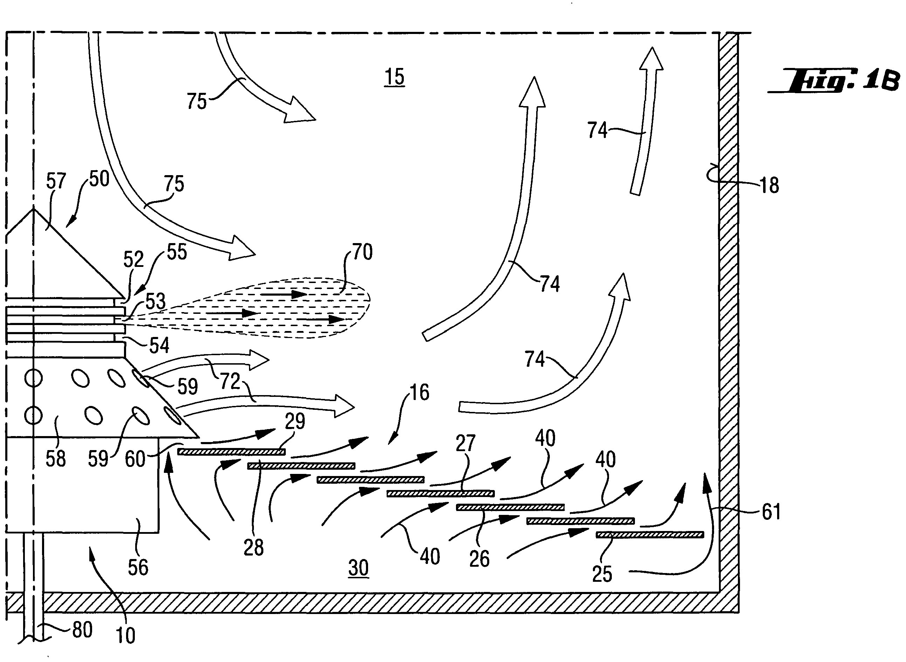

Im Bereich unterhalb des Mündungsspalts 54 ist eine kegelstumpfförmige Wand 58 vorhanden, die zahlreiche Öffnungen 59 aufweist. Wie aus der Fig. 1B zu erkennen ist, ruht die Unterseite der kegelstumpfförmigen Wand 58 auf der innersten Ringplatte 29 derart, dass zwischen der Unterseite der kegelstumpfförmigen Wand 58 und der darunter liegenden, mit dieser teilweise überlappenden Ringplatte 29 ein Schlitz 60 ausgebildet ist, durch den Prozessluft 40 hindurch treten kann.

Der äußere Ring 25 ist zu der Wand 18 beabstandet, so dass Prozessluft 40 in Richtung des mit dem Bezugzeichen 61 belegten Pfeiles mit einer überwiegend vertikalen Komponente in die Prozesskammer 15 eintreten kann und dadurch der durch die Schlitze 28 in die Prozesskammer 15 eintretenden Prozessluft 40 eine verhältnismäßig stark nach oben gerichtete Komponente verleiht.

Auf der rechten Hälfte der Fig. 1A ist dargestellt, welche Verhältnisse sich in einem eingelaufenen Zustand in der Vorrichtung 10 ausbilden.

Aus dem Mündungsspalt 53 tritt eine Sprühwolke 70 der Suspension aus, deren horizontale Spiegelebene in etwa parallel zur Bodenebene verläuft. Durch die Öffnungen 59 in der kegelstumpfförmigen Wand 58 durchtretende Luft, die beispielsweise Prozessluft 40 sein kann, bildet sich an der Unterseite der Sprühwolke 70 eine Stützluftströmung 72 aus. Durch die durch die zahlreichen Schlitze 28 hindurch tretende Prozessluft 40 bildet sich eine radiale Strömung in Richtung der Wand 18 aus, von der die Prozessluft 40 nach oben umgelenkt wird, wie das durch den mit dem Bezugzeichen 74 belegten Pfeil dargestellt ist. Von der umgelenkten Prozessluft 40 werden die Formkörper im Bereich der Wand 18 nach oben geführt. Die Prozessluft 40 und die zu behandelnden Katalysatorträger-Formkörper trennen sich dann voneinander, wobei die Prozessluft 40 durch Auslässe abgeführt wird, während sich die Formkörper radial gemäß der Pfeile 75 nach innen bewegen und aufgrund der Schwerkraft in Richtung des kegelförmigen Kopfes 57 der Ringspaltdüse 50 vertikal nach unten wandern. Dort werden die sich herabbewegenden Formkörper umgelenkt, auf die Oberseite der Sprühwolke 70 geleitet und dort mit dem versprühten Medium behandelt. Die besprühten Formkörper bewegen sich dann wieder in Richtung der Wand 18 und dabei voneinander weg, da nach Verlassen der Sprühwolke 70 an dem ringförmigen Mündungsspalt 53 den Formkörpern ein umfänglich größerer Raum zur Verfügung steht. Im Bereich der Sprühwolke 70 treffen die zu behandelnden Formkörper mit der versprühten Suspension zusammen und werden in Bewegungsrichtung in Richtung der Wand 18 bleibend voneinander weg bewegt und dabei sehr gleichmäßig und harmonisch mit der erwärmten Prozessluft 40 behandelt, d.h. getrocknet.

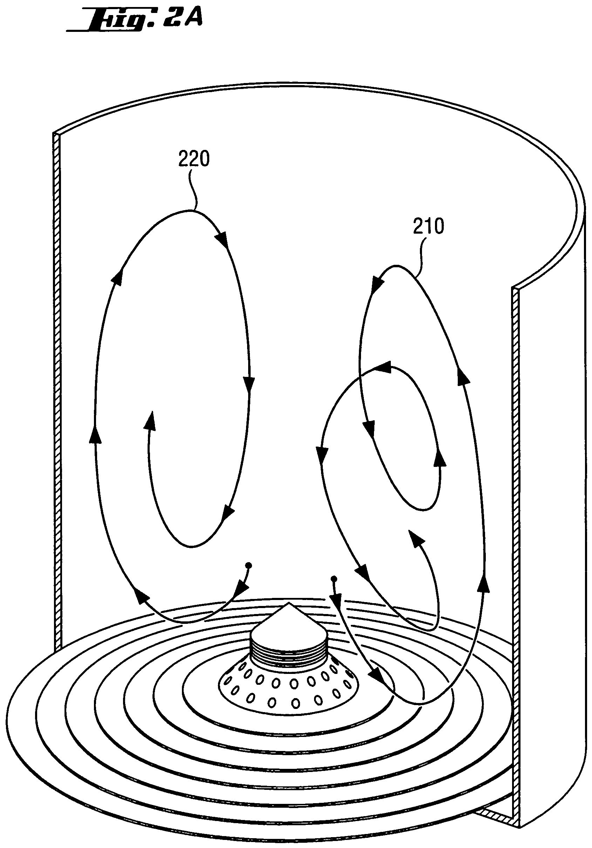

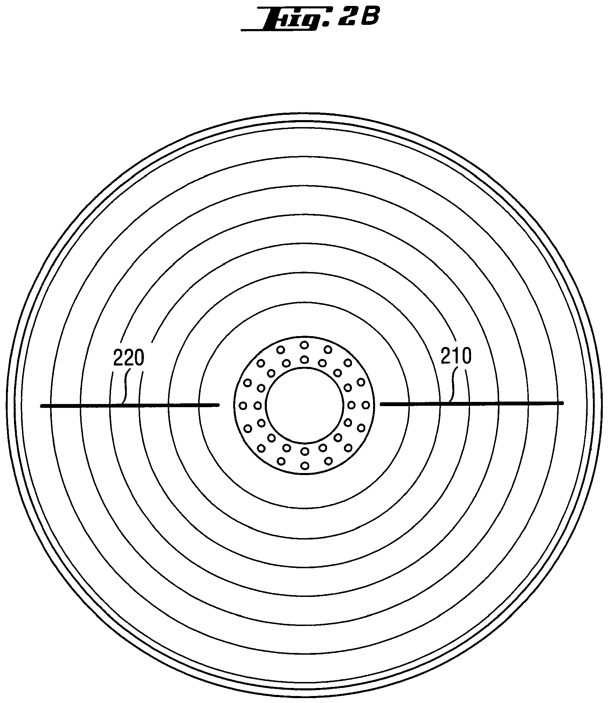

In der Figur 2A sind zwei mögliche Bewegungsbahnen zweier elliptisch umlaufender Katalysatorträger-Formkörper mittels der mit den Bezugszeichen 210 und 220 belegten Kurvenverläufe gezeigt. Die elliptische Bewegungsbahn 210 weist relativ große Änderungen in der Größe der Haupt- und Nebenachse auf im Vergleich zu einer idealen elliptischen Bahn. Die elliptische Bewegungsbahn 220 weist im Gegensatz dazu relativ kleine Änderung in der Größe der Haupt- und Nebenachse auf und beschreibt nahezu eine ideale elliptische Bahn ohne jegliche umfängliche (horizontale) Bewegungskomponente, wie aus der Figur 2B zu entnehmen ist.

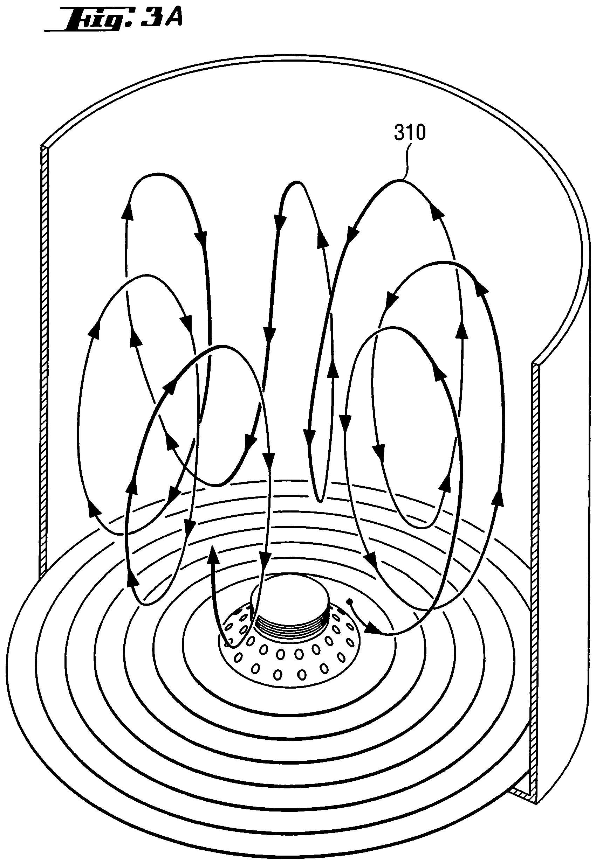

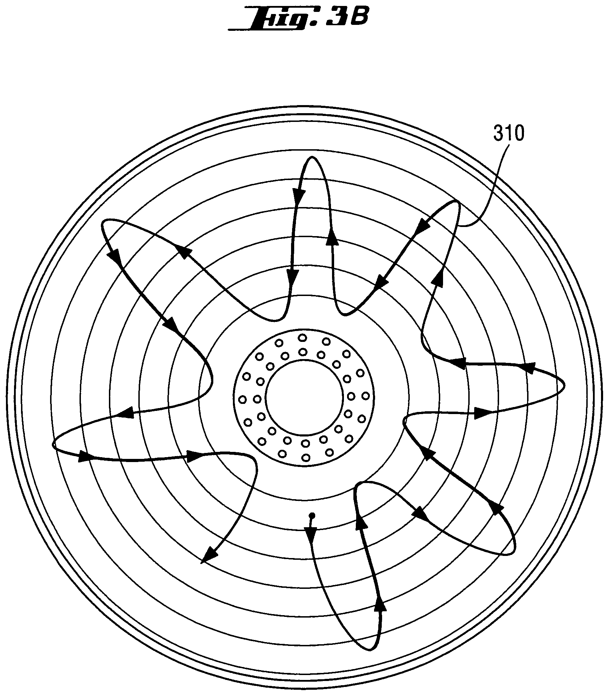

In der Figur 3A ist eine mögliche Bewegungsbahn eines toroidal umlaufenden Katalysatorträger-Formkörpers mittels des mit dem Bezugszeichen 310 belegten Kurvenverlaufs gezeigt. Die toroidal verlaufende Bewegungsbahn 310 beschreibt einen Ausschnitt der Oberfläche eines nahezu gleichförmigen Torus, dessen vertikaler Schnitt ellipsenförmig und dessen horizontaler Schnitt ringförmig ist. Die Figur 3B zeigt die Bewegungsbahn 310 in Draufsicht.

## Patentansprüche

1. Verfahren zur Herstellung von Schalenkatalysatoren durch Auftragen einer Washcoatsuspension auf eine Trägerstruktur, wobei das Verfahren unter Nutzung einer Vorrichtung (10) durchgeführt wird, die eingerichtet ist, mittels eines Prozessgases (40) ein Fließbett von Trägerstrukturen zu erzeugen, in welchem die Trägerstrukturen toroidal umlaufen, umfassend die Schritte des

    a) Beschickens der Vorrichtung (10) mit Trägerstrukturen und Erzeugens eines Trägerstruktur-Fließbettes mittels eines Prozessgases (40), wobei die Trägerstrukturen in dem Fließbett toroidal umlaufen;
    b) Imprägnierens der Trägerstrukturen mit einer Washcoatsuspension durch Besprühen der in dem Fließbett toroidal umlaufenden Trägerstrukturen mit der Washcoatsuspension;
    c) Trocknens der mit der Washcoatsuspension besprühten Trägerstrukturen;
    d) gegebenenfalls, Kalzinierens der mit den Feststoffanteilen der Washcoatsuspension beladenen Trägerstrukturen,

worin die Vorrichtung (10) eine Prozesskammer (15) mit einem Boden (16) umfasst, in dessen Mitte eine Ringspaltdüse (50) angeordnet ist,
die Prozesskammer (15) ferner eine Seitenwand (18) umfasst, wobei das Prozessgas (40) durch den Boden (16) der Prozesskammer (15), der vorzugsweise aus mehreren übereinander gelegten, sich einander überlappenden ringförmigen Leitplatten (25, 26, 27, 29) aufgebaut ist, und wobei die äußere Leitplatte (25) zu der Seitenwand (18) beabstandet ist, zwischen denen ringförmige Schlitze (28) ausgebildet sind, mit einer horizontalen, radial nach außen gerichteten Bewegungskomponente in die Prozesskammer (15) eingeführt wird zur Erzeugung des Trägerstruktur-Fließbettes,
und worin dem in die Prozesskammer (15) eingeführten Prozessgas (40) eine umfängliche Strömungskomponente auferlegt wird,
indem durch den Boden (16) der Prozesskammer (15) zusätzliches Prozessgas (61) mit einer schräg nach oben gerichteten Bewegungskomponente in die Prozesskammer (15) im Bereich der Seitenwand (18) der Prozesskammer (15) eingeführt wird.

2. Verfahren nach Anspruch 1, **dadurch gekennzeichnet, dass** dem in die Prozesskammer (15) eingeführten Prozessgas (40) die umfängliche Strömungskomponente mittels Leitelementen auferlegt wird, die zwischen den ringförmigen Leitplatten (25, 26, 27, 29) angeordnet sind.

3. Verfahren nach einem der Ansprüche 1 oder 2, **dadurch gekennzeichnet, dass** die Ringspaltdüse (50) eine Sprühwolke (70) versprüht, die parallel zur Ebene des Bodens (16) verläuft.

4. Verfahren nach Anspruch 3, **dadurch gekennzeichnet, dass** die Mündung (55) der Ringspaltdüse (50) in das Fließbett eingebettet ist.

5. Verfahren nach Anspruch 3 oder 4, **dadurch gekennzeichnet, dass** an der Unterseite der Sprühwolke (70) ein Gasstützpolster (72) bewerkstelligt wird.

6. Verfahren nach einem der voranstehenden Ansprüche, **dadurch gekennzeichnet, dass** die Trägerstruktur aus einem nicht-porösen Material oder Materialgemisch gebildet ist, vorzugsweise aus Steatit, Quarz ($SiO_2$), Porzellan, Magnesiumoxid, Zinndioxid, Siliciumcarbid, Rutil, Tonerde ($Al_2O_3$), Aluminiumsilicat, Magnesiumsilicat, Zirkoniumsilicat oder aus Cersilicat oder aus Mischungen von zwei oder mehr der vorstehenden Materialien.

7. Verfahren nach einem der voranstehenden Ansprüche, **dadurch gekennzeichnet, dass** die Trägerstruktur eine Härte von größer/gleich 20 N aufweist, vorzugsweise eine von größer/gleich 30 N, weiter bevorzugt eine von größer/gleich 40 N und am meisten bevorzugt eine von größer/gleich 80 N.

8. Verfahren nach einem der voranstehenden Ansprüche, **dadurch gekennzeichnet, dass** das Prozessgas (40) ausgewählt ist aus der Gruppe bestehend aus Luft, Sauerstoff, Stickstoff und den Edelgasen, vorzugsweise Helium und Argon.

9. Verfahren nach einem der voranstehenden Ansprüche, **dadurch gekennzeichnet, dass** das Prozessgas (40) erwärmt wird, vorzugsweise auf eine Temperatur von größer/gleich 40 °C, bevorzugt auf eine Temperatur von größer/gleich 60 °C, weiter bevorzugt auf eine Temperatur von größer/gleich 70 °C und am meisten bevorzugt auf eine Temperatur von 60 bis 100 °C.

10. Verfahren nach einem der voranstehenden Ansprüche, **dadurch gekennzeichnet, dass** das Prozessgas (40) vor der Einführung in die Prozesskammer (15) mit dem Suspensionsmittel der Washcoatsuspension, beispielsweise Wasser, angereichert wird, vorzugsweise in einem Bereich von 10 bis 50 % des Sättigungsdampfdrucks.

11. Verfahren nach einem der voranstehenden Ansprüche, **dadurch gekennzeichnet, dass** die Trägerstruktur als Kugel, Zylinder, Lochzylinder, Trilobus, Tetralobus, Ring, Donut, Stern, Wagenrad oder als Strang, vorzugsweise als Rippstrang oder Sternstrang, ausgebildet ist.

12. Verfahren nach einem der voranstehenden Ansprüche, **dadurch gekennzeichnet, dass** die Washcoatsuspension $TiO_2$, $V_2O_5$ sowie $Sb_2O_3$ in partikulärer Form enthält.

13. Verfahren nach Anspruch 12, **dadurch gekennzeichnet, dass** die Suspension ferner eine Phosphatverbindung, eine Alkali- und/oder Erdalkaliverbindung sowie ein organisches und/oder anorganisches Bindemittel umfasst.

14. Verfahren nach Anspruch 12 oder 13, **dadurch gekennzeichnet, dass** die Washcoatsuspension auf einen Ring als Trägerstruktur aufgesprüht wird.

15. Verfahren nach einem der voranstehenden Ansprüche 1 bis 11, **dadurch gekennzeichnet, dass** die Washcoatsuspension Eisenmolybdat enthält.

16. Verfahren nach einem der voranstehenden Ansprüche 1 bis 11, **dadurch gekennzeichnet, dass** die Washcoatsuspension ein Mischoxid von Mo, V und W, oder Mo, V und Nb oder Ta sowie gegebenenfalls Te oder Sb enthält, das vorzugsweise mit Cu, Mn oder Fe dotiert ist.

17. Charge einer Vielzahl von Schalenkatalysatoren hergestellt nach einem Verfahren der voranstehenden Ansprüche, jeder Schalenkatalysator umfassend einen Formkörper, auf dem eine ein metalloxidisches Material umfassende Schale aufgetragen ist, wobei das Verhältnis der Standardabweichung der Schalendicken der Schalenkatalysatoren der Charge zum Mittelwert der Schalendicken der Schalenkatalysatoren der Charge 3-15% ist.

**Claims**

1. Method for producing shell catalysts by applying a washcoat suspension to a support structure, wherein the method is carried out using a device (10) which is set up to produce by means of a process gas (40) a fluid bed of support structures, in which the support structures circulate toroidally, said method comprising the steps of

a) charging the device (10) with support structures and producing a support-structure fluid bed by means of a process gas (40), wherein the support structures circulate in the fluid bed toroidally;
b) impregnating the support structures with a washcoat suspension by spraying the support structures circulating toroidally in the fluid bed with the washcoat suspension;
c) drying the support structures sprayed with the washcoat suspension;
d) optionally calcining the support structures loaded with the solids contents of the washcoat suspension,

wherein the device (10) comprises a process chamber (15) with a bottom (16) in the centre of which bottom an annular gap nozzle (50) is arranged, the process chamber (15) furthermore comprises a side wall (18), wherein the process gas (40) is fed, with a horizontal movement component aligned radially outwards, into the process chamber (15) through the bottom (16) of the process chamber (15), the bottom being preferably constructed of several overlapping annular guide plates laid one over the other (25, 26, 27, 29) between which annular slots (28) are formed, and wherein the outer guide plate (25) is at a distance from the wall (18), in order to produce the support-structure fluid bed, and wherein the process gas (40) fed into the process chamber (15) is subjected to a circumferential flow component, by feeding additional process gas (61), with a movement component aligned diagonally upwards, through the bottom (16) of the process chamber (15) into the process chamber (15), in the area of the side wall (18) of the process chamber (15).

2. Method according to claim 1, **characterized in that** the process gas (40) fed into the process chamber (15) is subjected to the circumferential flow component by means of guide elements which are arranged between the annular guide plates (25, 26, 27, 29).

3. Method according to one of claims 1 or 2, **characterized in that** the annular gap nozzle (50) atomizes a spray cloud (70) which runs parallel to the plane of the bottom (16).

4. Method according to claim 3, **characterized in that** the mouth (55) of the annular gap nozzle (50) is embedded into the fluid bed.

5. Method according to claim 3 or 4, **characterized in that** a gas support cushion (72) is produced on the underside of the spray cloud (70).

6. Method according to one of the previous claims, **characterized in that** the support structure is formed from a non-porous material or material mixture, preferably from steatite, quartz ($SiO_2$), porcelain, magnesium oxide, tin dioxide, silicon carbide, rutile, alumina ($Al_2O_3$), aluminium silicate, magnesium silicate, zirconium silicate or from cerium silicate or from mixtures of two or more of the above materials.

7. Method according to one of the previous claims, **characterized in that** the support structure has a hardness greater than/equal to 20 N, preferably greater than/equal to 30 N, further preferably greater than/equal to 40 N and most preferably greater than/equal to 80 N.

8. Method according to one of the previous claims, **characterized in that** the process gas (40) is selected from the group consisting of air, oxygen, nitrogen and the noble gases, preferably helium and argon.

9. Method according to one of the previous claims, **characterized in that** the process gas (40) is heated, preferably to a temperature of more than/equal to 40°C, by preference to a temperature of more than/equal to 60°C, further preferably to a temperature of more than/equal to 70°C and most preferably to a temperature of 60 to 100°C.

10. Method according to one of the previous claims, **characterized in that** the process gas (40) is enriched, before being fed into the process chamber (15), with the suspending agent of the washcoat suspension, for example water, preferably in a range of 10 to 50% of the saturation vapour pressure.

11. Method according to one of the previous claims, **characterized in that** the support structure is formed as sphere, cylinder, perforated cylinder, trilobe, tetralobe, ring, doughnut, star, cartwheel or as strand, preferably as ribbed strand or star strand.

12. Method according to one of the previous claims, **characterized in that** the washcoat suspension contains $TiO_2$, $V_2O_5$ and $Sb_2O_3$ in particulate form.

13. Method according to claim 12, **characterized in that** the suspension furthermore comprises a phosphate compound, an alkali and/or alkaline earth compound, and an organic and/or inorganic binding agent.

14. Method according to claim 12 or 13, **characterized in that** the washcoat suspension is sprayed onto a ring as support structure.

15. Method according to one of the previous claims 1 to 11, **characterized in that** the washcoat suspension contains iron molybdate.

16. Method according to one of the previous claims 1 to 11, **characterized in that** the washcoat suspension contains a mixed oxide of Mo, V and W, or Mo, V and Nb, or Ta as well as optionally Te or Sb, which is preferably doped with Cu, Mn or Fe.

17. Batch of a plurality of shell catalysts produced according to a method of the previous claims, each shell catalyst comprising a shaped body to which a shell comprising a metal-oxide material is applied, wherein the ratio of the standard deviation of the shell thicknesses of the shell catalysts of the batch to the mean value of the shell thicknesses of the shell catalysts of the batch is 3 to 15%.

**Revendications**

1. Procédé de fabrication de catalyseurs à coque par application d'une suspension d'imprégnateur sur une structure porteuse, dans lequel le procédé est réalisé en utilisant un dispositif (10) qui est agencé pour générer un lit fluidisé de structures porteuses au moyen d'un gaz de processus (40) dans lequel les structures porteuses circulent de manière toroïdale, comprenant les étapes de

     a) alimentation du dispositif (10) avec des structures porteuses et génération d'un lit fluidisé de structures porteuses au moyen d'un gaz de processus (40), dans lequel les structures porteuses circulent de manière toroïdale dans le lit fluidisé ;
     b) imprégnation des structures porteuses avec une suspension d'imprégnateur par pulvérisation des structures porteuses circulant de manière toroïdale dans le lit fluidisé avec la suspension d'imprégnateur ;

c) séchage des structures porteuses pulvérisées avec la suspension d'imprégnateur ;

d) éventuellement, calcination des structures porteuses chargées avec les fractions solides de la suspension d'imprégnateur,

dans lequel le dispositif (10) comprend une chambre de processus (15) avec un fond (16) dans le milieu duquel une tuyère à fente annulaire (50) est disposée,

la chambre de processus (15) comprend en outre une paroi latérale (18), dans lequel le gaz de processus (40) est introduit dans la chambre de processus (15) à travers le fond (16) de la chambre de processus (15) qui est de préférence constitué de plusieurs plaques de guidage annulaires (25, 26, 27, 29) situées les unes au-dessus des autres, se chevauchant les unes les autres, et dans lequel la plaque de guidage extérieure (25) est écartée de la paroi latérale (18), entre lesquelles des fentes annulaires (28) sont réalisées, avec un composant de mouvement horizontal, orienté radialement vers l'extérieur pour la génération du lit fluidisé de structures porteuses,

et dans lequel un composant d'écoulement circonférentiel est appliqué au gaz de processus (40) introduit dans la chambre de processus (15),

en ce que du gaz de processus supplémentaire (61) est introduit dans la chambre de processus (15) dans la région de la paroi latérale (18) de la chambre de processus (15) à travers le fond (16) de la chambre de processus (15) avec un composant de mouvement orienté en biais vers le haut.

2. Procédé selon la revendication 1, **caractérisé en ce que** le composant d'écoulement circonférentiel est appliqué au gaz de processus (40) introduit dans la chambre de processus (15) au moyen d'éléments de guidage qui sont disposés entre les plaques de guidage annulaires (25, 26, 27, 29).

3. Procédé selon une des revendications 1 ou 2, **caractérisé en ce que** la tuyère à fente annulaire (50) pulvérise un nuage de pulvérisation (70) qui s'étend parallèlement au plan du fond (16).

4. Procédé selon la revendication 3, **caractérisé en ce que** la bouche (55) de la tuyère à fente annulaire (50) est encastrée dans le lit fluidisé.

5. Procédé selon la revendication 3 ou 4, **caractérisé en ce qu'**un matelas de gaz (72) est produit sur le côté inférieur du nuage de pulvérisation (70).

6. Procédé selon une des revendications précédentes, **caractérisé en ce que** la structure porteuse est formée d'un matériau ou mélange de matériaux non poreux, de préférence de stéatite, quartz ($SiO_2$), porcelaine, oxyde de magnésium, dioxyde d'étain, carbure de silicium, rutile, alumine ($Al_2O_3$), silicate d'aluminium, silicate de magnésium, silicate de zirconium ou de silicate de cérium ou de mélanges de deux des matériaux précédents ou plus.

7. Procédé selon une des revendications précédentes, **caractérisé en ce que** la structure porteuse présente une dureté supérieure/égale à 20 N, de préférence une dureté supérieure/égale à 30 N, de manière davantage préférée une dureté supérieure/égale à 40 N et de manière préférée entre toutes une dureté supérieure/égale à 80 N.

8. Procédé selon une des revendications précédentes, **caractérisé en ce que** le gaz de processus (40) est sélectionné parmi le groupe constitué de l'air, l'oxygène, l'azote et des gaz nobles, de préférence l'hélium et l'argon.

9. Procédé selon une des revendications précédentes, **caractérisé en ce que** le gaz de processus (40) est chauffé, de préférence à une température supérieure/égale à 40°C, de manière préférée à une température supérieure/égale à 60°C, de manière davantage préférée à une température supérieure/égale à 70°C et de manière préférée entre toutes à une température de 60 à 100°C.

10. Procédé selon une des revendications précédentes, **caractérisé en ce que** le gaz de processus (40) est enrichi avant l'introduction dans la chambre de processus (15) avec l'agent de suspension de la suspension d'imprégnateur, par exemple de l'eau, de préférence dans une plage de 10 à 50 % de la pression de vapeur saturante.

11. Procédé selon une des revendications précédentes, **caractérisé en ce que** la structure porteuse est réalisée en tant que bille, cylindre, cylindre perforé, trilobe, tétralobe, anneau, tore, étoile, roue ou en tant que cordon, de préférence en tant que cordon nervuré ou cordon étoilé.

12. Procédé selon une des revendications précédentes, **caractérisé en ce que** la suspension d'imprégnateur contient du $TiO_2$, $V_2O_5$ ainsi que $Sb_2O_3$ sous forme particulaire.

13. Procédé selon la revendication 12, **caractérisé en ce que** la suspension comprend en outre un composé de phosphate, un composé alcalin et/ou alcalino-terreux ainsi qu'un liant organique et/ou inorganique.

**14.** Procédé selon la revendication 12 ou 13, **caractérisé en ce que** la suspension d'imprégnateur est pulvérisée sur un anneau comme structure porteuse.

**15.** Procédé selon une des revendications précédentes 1 à 11, **caractérisé en ce que** la suspension d'imprégnateur contient du molybdate de fer.

**16.** Procédé selon une des revendications précédentes 1 à 11, **caractérisé en ce que** la suspension d'imprégnateur contient un oxyde mixte de Mo, V et W, ou Mo, V et Nb ou Ta ainsi qu'éventuellement Te ou Sb, qui est de préférence dopé avec Cu, Mn ou Fe.

**17.** Charge d'une pluralité de catalyseurs à coque fabriqués selon un procédé des revendications précédentes, chaque catalyseur à coque comprenant un corps moulé sur lequel une coque comprenant un matériau d'oxyde métallique est appliquée, dans laquelle le rapport de l'écart-type des épaisseurs de coque des catalyseurs à coque de la charge sur la valeur moyenne des épaisseurs de coque des catalyseurs à coque de la charge est de 3 à 15 %.

Fig. 1A

Fig. 1B

EP 2 160 241 B1

Fig. 2A

16

Fig. 2B

**Fig. 3A**

310

Fig. 3B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005030380 A
- DE 102005055827
- US 4259211 A
- WO 2006027009 A1
- DE 10248116 B3
- EP 0370167 A1
- EP 0436787 B1
- DE 19904147 A1
- DE 202005003791 U1
- EP 1674156 A1